# FASCICULE DE BREVET EUROPEEN

(11) **EP 2 184 050 B1**
(45) Date de publication et mention de la délivrance du brevet: **11.01.2017**
(21) Numéro de dépôt: 09175301.2
(22) Date de dépôt: 06.11.2009
(51) Int. Cl.: A61K 8/41, A61K 8/49, A61Q 5/10

(54) **Composition comprenant la 2,3-diamino-6,7-dihydro-1H,5H-pyrazolo[1,2-a]pyrazol-1-one, le 4,5-diamino 1-(ß-hydroxyethyl) pyrazole et le 2-chloro 6-methyl 3-amino phenol**
Zusammensetzung enthaltend 2,3-diamino-6,7-dihydro-1H,5H-pyrazolo[1,2-a]pyrazol-1-one, 4,5-diamino-1-(ß-hydroxyethyl)pyrazole and 2 chloro 6 methyl 3 aminophenol
Composition comprising 2,3-diamino-6,7-dihydro-1H,5H-pyrazolo[1,2-a]pyrazol-1-one, 4,5-diamino-1-(ß-hydroxyethyl)pyrazole and 2 chloro 6 methyl 3 aminophenol

(30) Priorité: 06.11.2008 FR 0857539
(43) Date de publication de la demande: 12.05.2010
(73) Titulaire: L'Oréal, 75008 Paris (FR)
(72) Inventeur: Deconinck, Gautier, 95210, Saint Gratien (FR); Saunier, Jean-Baptiste, 75014, Paris (FR); Desenne, Patricia, 92270, Bois Colombes (FR)
(74) Mandataire: Prevel, Estelle Nicole

(56) Documents cités:
- EP-A- 1 764 082
- EP-A- 1 870 083
- EP-A- 1 927 376
- EP-A- 1 927 377
- FR-A- 2 886 141

## Description

L'invention a pour objet une composition pour la teinture des fibres kératiniques, et en particulier des fibres kératiniques humaines telles que les cheveux, comprenant la 2,3-diamino-6,7-dihydro-1H,5H-pyrazolo[1,2-a]pyrazol-1-one à titre de première base d'oxydation, le 4,5-diamino 1-(β-hydroxyéthyl) pyrazole à titre de deuxième base d'oxydation et le 2-chloro 6-méthyl 3-amino phénol à titre de premier coupleur.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, d'uniformité de la couleur et de la ténacité aux agents extérieurs.

L'utilisation de ces bases à pH neutre ne permet pas d'atteindre une gamme de nuances variées, en particulier pour les nuances chaudes telles que les rouges et les orangés. Le document EP1927376 divulgue les compositions de coloration de pH acide comprenant la 2,3-diamino-6,7-dihydro-1H,5H-pyrazolo[1,2-a]pyrazol-1-one.

Le but de la présente invention est de fournir de nouvelles compositions de coloration des fibres kératiniques qui permettent d'obtenir une coloration avec des nuances rouges et / ou cuivrées, et en particulier des nuances intermédiaires rouges-cuivrées et cuivrées-rouges, intenses, puissantes, chromatiques, esthétiques, peu sélectives et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié :
- au moins une première base d'oxydation choisie parmi la 2,3-diamino-6,7-dihydro-1H,5H-pyrazolo[1,2-a]pyrazol-1-one de formule (I) suivante et ses sels d'addition : 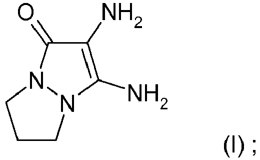
- au moins une deuxième base d'oxydation choisie parmi le 4,5-diamino 1-(β-hydroxyéthyl) pyrazole et ses sels d'addition ; et
- au moins un coupleur choisi parmi le 2-chloro 6-méthyl 3-amino phénol et ses sels d'addition.

La présente invention permet d'obtenir une coloration des fibres kératiniques aux nuances rouges et / ou cuivrées intenses et / ou chromatiques. Les nuances intermédiaires cuivrées-rouges et rouges-cuivrées obtenues sont particulièrement esthétiques avec une forte intensité et / ou chromaticité.

La présente invention permet également d'obtenir des colorations particulièrement peu sélectives et résistant bien aux diverses agressions que peuvent subir les cheveux tels que les shampooings, la lumière, la sueur et les déformations permanentes. Elle permet de plus d'obtenir des colorations intenses à pH neutre.

Un autre objet de l'invention est un procédé de teinture des fibres kératiniques mettant en oeuvre la composition de la présente invention, ainsi que l'utilisation de cette composition pour la teinture des fibres kératiniques.

L'invention a enfin pour objet un kit de coloration comprenant d'une part une composition de coloration contenant la 2,3-diamino-6,7-dihydro-1H,5H-pyrazolo[1,2-a]pyrazol-1-one ou l'un de ses sels à titre de première base d'oxydation, le 4,5-diamino 1-(β-hydroxyéthyl) pyrazole ou l'un de ses sels à titre de deuxième base d'oxydation et le 2-chloro 6-méthyl 3-amino phénol ou l'un de ses sels à titre de coupleur et d'autre part une composition contenant un ou plusieurs agents oxydants.

Selon un mode de réalisation particulier, dans la composition conforme à l'invention, le rapport molaire de la concentration en 2-chloro 6-méthyl 3-amino phénol ou l'un ses sels d'addition au total des concentrations des première et deuxième bases d'oxydation est inférieur ou égal à 1, de préférence va de 0,05 à 1, et encore plus préférentiellement de 0,1 à 0,8.

Il n'est pas exclu bien que non préféré que les compositions de l'invention contiennent la première base d'oxydation et / ou la seconde base d'oxydation respectivement sous forme d'un mélange de plusieurs sels ou d'un mélange de composé non salifié avec un ou plusieurs sels.

A moins d'une indication différente, les bornes des gammes de valeurs qui sont données dans le cadre de la présente invention sont incluses dans ces gammes.

La composition tinctoriale de l'invention peut contenir une ou plusieurs bases d'oxydation différentes de celles utiles dans la présente invention et conventionnellement utilisées pour la teinture de fibres kératiniques.

La composition de la présente invention peut par exemple comprendre au moins une base d'oxydation additionnelle choisie parmi les para-phénylènediamines, les bis-phénylalkylènediamines, les para-aminophénols, les bis-para-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one, du 4,5-diamino 1-(β-hydroxyéthyl) pyrazole et de leurs sels d'addition.

Parmi les para-phénylènediamines, on peut citer, à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition sont particulièrement préférées.

Parmi les bis-phénylalkylènediamines, on peut citer, à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition.

Parmi les ortho-aminophénols, on peut citer, à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer, à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Dans une variante de l'invention, la composition conforme à l'invention comprend une ou plusieurs bases d'oxydation additionnelles choisies parmi les para-aminophénols et les para-phénylènediamines. Parmi les para-aminophénols, le para-aminophénol, le 3-méthyl 4-amino phénol et leurs sels d'addition sont particulièrement préférés. Parmi les para-phénylènediamines, la para-phénylènediamine, la para-toluènediamine et la N,N-bis-(β-hydroxyéthyl) para-phénylènediamine et leurs sels d'addition sont particulièrement préférés.

La composition tinctoriale de l'invention peut contenir des coupleurs additionnels différents de celui utile dans la présente invention et conventionnellement utilisés pour la teinture de fibres kératiniques.

La composition de la présente invention peut par exemple comprendre un ou plusieurs coupleurs additionnels choisis parmi les méta-phénylènediamines, les méta-aminophénols différents du 2-chloro 6-méthyl 3-amino phénol et de ses sels d'addition, les méta-diphénols, les coupleurs naphtaléniques, les coupleurs hétérocycliques.

A titre d'exemples de coupleurs additionnels, on peut citer le 3-amino phénol, le 2-méthyl 5-aminophénol, le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène et leurs sels d'addition.

De préférence, la composition conforme à l'invention comprend au moins un coupleur additionnel choisi parmi les méta-aminophénols autres que celui de l'invention. Encore plus préférentiellement, la composition conforme à l'invention comprend au moins un coupleur additionnel choisi parmi le 2-méthyl 5-aminophénol, le 2-méthyl 5-(ß-hydroxyéthylamino) phénol et leurs sels d'addition.

La ou les bases d'oxydation y compris celles de l'invention présentes dans la composition de l'invention sont en général présentes chacune en quantité comprise entre 0,001 à 10 % en poids environ du poids total de la composition tinctoriale, de préférence entre 0,005 et 6 %.

Le ou les coupleurs y compris celui de l'invention présents dans la composition de l'invention sont en général présents chacun en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les alkyl(C₁-C₄)sulfonates, et en particulier les méthylsulfonates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'un ou plusieurs solvants organiques. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymériques anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 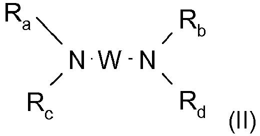 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un ou plusieurs agents oxydants. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, un ou plusieurs agents oxydants, ce ou ces agents oxydants étant présents en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition telle que définie précédemment.

Les exemples qui suivent servent à illustrer l'invention sans toutefois présenter un caractère limitatif.

### EXEMPLE 1

La composition de coloration suivante a été réalisée (quantités exprimées en produit commercial tel quel sauf indication contraire) :

| | |
|---|---|
| Alcool laurique à 12 OE | 7 g |
| Alcool décylique à 3 OE | 10 g |
| Alcool oléocétylique à 30 OE | 4 g |
| Alcool cétylstéarylique | 11,5 g |
| Acide laurique | 3 g |
| Distéarate de glycol | 2 g |
| Aerosil R 972 (DEGUSSA) | 1,2 g |
| Carbopol 980 (LUBRIZOL) | 0,4 g |
| Propylène glycol | 10 g |
| Monoéthanolamine | 1,6 g |
| Mexomère PO (CHIMEX) | 3 g MA |
| Merquat 280 (NALCO) | 2,2 g MA |
| Agent séquestrant, anti-oxydant, réducteur, parfum | q.s. |
| Ammoniaque (20 % de NH₃) | 10 g |
| 2,3-diamino-6,7-dihydro-1H,5H-pyrazolo[1,2-a]pyrazol-1-one, 2 CH₃-SO₃H | 0,3 g |
| 4,5-diamino 1-(β-hydroxyéthyl) pyrazole, sulfate | 1,7 g |
| 2-chloro 6-méthyl 3-amino phénol | 0,3 g |
| Para-phénylènediamine | 0,42 g |
| Para-aminophénol | 0,2 g |
| 2-méthyl 5-aminophénol | 1,4 g |
| Eau | q.s.p. 100 g |

| | |
|---|---|
| MA = Matières actives | |

Au moment de l'emploi, 1 partie en poids de la composition de coloration décrite ci-dessus est mélangée avec 1,5 parties en poids d'une solution de peroxyde d'hydrogène à 20 volumes à pH 2,2. On obtient un pH final proche de 9,6.

Le mélange obtenu est appliqué sur des mèches de cheveux gris à 90 % de blancs. Après 30 minutes de pose à température ambiante, les mèches sont rincées, lavées avec un shampooing standard, rincées à nouveau puis séchées.

La coloration capillaire est évaluée de manière visuelle. On obtient une nuance blond rouge cuivrée peu sélective et chromatique.

### EXEMPLE 2

La composition de coloration suivante a été réalisée (quantités exprimées en produit commercial tel quel sauf indication contraire) :

| | |
|---|---|
| Alcool laurique à 12 OE | 7 g |
| Alcool décylique à 3 OE | 10 g |
| Alcool oléocétylique à 30 OE | 4 g |
| Alcool cétylstéarylique | 11,5 g |
| Acide laurique | 3 g |
| Distéarate de glycol | 2 g |
| Aerosil R 972 (DEGUSSA) | 1,2 g |
| Carbopol 980 (LUBRIZOL) | 0,4 g |
| Propylène glycol | 10 g |
| Monoéthanolamine | 1,6 g |
| Mexomère PO (CHIMEX) | 3 g MA |
| Merquat 280 (NALCO) | 2,2 g MA |
| Agent séquestrant, anti-oxydant, réducteur, parfum | q.s. |
| Ammoniaque (20 % de NH₃) | 10 g |
| 2,3-diamino-6,7-dihydro-1H,5H-pyrazolo[1,2-a]pyrazol-1-one, 2 CH₃-SO₃H | 0,3 g |
| 4,5-diamino 1-(β-hydroxyéthyl) pyrazole, sulfate | 1,7 g |
| 2-chloro 6-méthyl 3-amino phénol | 0,3 g |
| 2-méthyl 5-aminophénol | 1,0 g |
| Eau | q.s.p. 100 g |

| | |
|---|---|
| MA = Matières actives | |

Au moment de l'emploi, 1 partie en poids de la composition de coloration décrite ci-dessus est mélangée avec 1,5 parties en poids d'une solution de peroxyde d'hydrogène à 20 volumes à pH 2,2. On obtient un pH final proche de 9,6.

Le mélange obtenu est appliqué sur des mèches de cheveux gris à 90 % de blancs. Après 30 minutes de pose à température ambiante, les mèches sont rincées, lavées avec un shampooing standard, rincées à nouveau puis séchées.

La coloration capillaire est évaluée de manière visuelle. On obtient une nuance rouge cuivrée peu sélective et chromatique.

## Revendications

1. Composition de coloration des fibres kératiniques comprenant, dans un milieu approprié :
• au moins une première base d'oxydation choisie parmi la 2,3-diamino-6,7-dihydro-1H,5H-pyrazolo[1,2-a]pyrazol-1-one et ses sels d'addition ;
• au moins une deuxième base d'oxydation choisie parmi le 4,5-diamino 1-(β-hydroxyéthyl) pyrazole et ses sels d'addition ; et
• au moins un coupleur choisi parmi le 2-chloro 6-méthyl 3-amino phénol et ses sels d'addition.

2. Composition selon la revendication 1 dans laquelle le rapport molaire de la concentration en 2-chloro 6-méthyl 3-amino phénol ou l'un ses sels d'addition au total des concentrations des première et deuxième bases d'oxydation est inférieur ou égal à 1.

3. Composition selon l'une des revendications 1 et 2 comprenant une ou plusieurs bases d'oxydation additionnelles choisies parmi les para-aminophénols et les para-phénylènediamines.

4. Composition selon la revendication 3 dans laquelle le ou les para-aminophénols sont choisis parmi le para-aminophénol, le 3-méthyl 4-amino phénol et leurs sels d'addition.

5. Composition selon la revendication 3 dans laquelle le ou les para-phénylènediamines sont choisies parmi la para-phénylènediamine, la para-toluènediamine, la N,N-bis-(β-hydroxyéthyl) para-phénylènediamine et leurs sels d'addition.

6. Composition selon l'une quelconque des revendications précédentes comprenant un ou plusieurs coupleurs additionnels.

7. Composition selon la revendication 6 dans laquelle le ou les coupleurs additionnels sont choisis parmi le 2-méthyl 5-aminophénol, le 2-méthyl 5-(ß-hydroxyéthylamino) phénol et leurs sels d'addition.

8. Composition selon l'une quelconque des revendications précédentes dans laquelle la ou les bases d'oxydation sont présentes chacune en une quantité comprise entre 0,001 à 10 % en poids environ du poids total de la composition.

9. Composition selon l'une quelconque des revendications précédentes dans laquelle le ou les coupleurs sont présents chacun en une quantité comprise entre 0,001 et 10 % en poids environ du poids total de la composition.

10. Composition selon l'une quelconque des revendications précédentes comprenant de plus un ou plusieurs agents oxydants.

11. Procédé de teinture des fibres kératiniques dans lequel une composition telle que définie dans l'une quelconque des revendications 1 à 9 est appliquée sur les fibres kératiniques en présence d'un ou plusieurs agents oxydants pendant un temps suffisant pour développer la coloration désirée.

12. Dispositif à plusieurs compartiments dans lequel un premier compartiment contient une composition tinctoriale telle que définie dans l'une quelconque des revendications 1 à 9 et un deuxième compartiment contient un ou plusieurs agents oxydants.

13. Utilisation pour la teinture d'oxydation des fibres kératiniques d'une composition telle que définie dans l'une quelconque des revendications 1 à 10.

## Patentansprüche

1. Zusammensetzung zum Färben von Keratinfasern, umfassend in einem geeigneten Medium:
• mindestens eine erste Oxidationsbase, die aus 2,3-Diamino-6,7-dihydro-1H,5H-pyrazolo[1,2-a]-pyrazol-1-on und Additionssalzen davon ausgewählt ist;
• mindestens eine zweite Oxidationsbase, die aus 4,5-Diamino-1-(β-hydroxyethyl)pyrazol und Additionssalzen davon ausgewählt ist; und
• mindestens einen Kuppler, der aus 2-Chlor-6-methyl-3-aminophenol und Additionssalzen davon ausgewählt ist.

2. Zusammensetzung nach Anspruch 1, wobei das Molverhältnis der Konzentration von 2-Chlor-6-methyl-3-aminophenol oder einem Additionssalz davon zur Summe der Konzentrationen der ersten und zweiten Oxidationsbase kleiner gleich 1 ist.

3. Zusammensetzung nach einem der Ansprüche 1 und 2, umfassend eine oder mehrere zusätzliche Oxidationsbasen, die aus para-Aminophenolen und para-Phenylendiaminen ausgewählt sind.

4. Zusammensetzung nach Anspruch 3, wobei das bzw. die para-Aminophenole aus para-Aminophenol, 3-Methyl-4-aminophenol und Additionssalzen davon ausgewählt sind.

5. Zusammensetzung nach Anspruch 3, wobei das bzw. die para-Phenylendiamine aus para-Phenylendiamin, para-Toluoldiamin, N,N-Bis(β-hydroxyethyl)-para-phenylendiamin und Additionssalzen davon ausgewählt sind.

6. Zusammensetzung nach einem der vorhergehenden Ansprüche, umfassend einen oder mehrere zusätzliche Kuppler.

7. Zusammensetzung nach Anspruch 6, wobei der bzw.
die zusätzlichen Kuppler aus 2-Methyl-5-aminophenol, 2-Methyl-5-(β-hydroxyethylamino)phenol und Additionssalzen davon ausgewählt sind.

8. Zusammensetzung nach einem der vorhergehenden Ansprüche, wobei die Oxidationsbase bzw. Oxidationsbasen jeweils in einer Menge zwischen ungefähr 0,001 und 10 Gew.-%, bezogen auf das Gesamtgewicht der Zusammensetzung, vorliegen.

9. Zusammensetzung nach einem der vorhergehenden Ansprüche, wobei der bzw. die Kuppler jeweils in einer Menge zwischen ungefähr 0,001 und 10 Gew.-%, bezogen auf das Gesamtgewicht der Zusammensetzung, vorliegen.

10. Zusammensetzung nach einem der vorhergehenden Ansprüche, die außerdem ein oder mehrere Oxidationsmittel umfasst.

11. Verfahren zum Färben von Keratinfasern, **dadurch gekennzeichnet, dass** man auf die Keratinfasern eine Zusammensetzung gemäß einem der Ansprüche 1 bis 9 in Gegenwart eines oder mehrerer Oxidationsmittel über einen zur Entwicklung der gewünschten Färbung ausreichenden Zeitraum aufbringt.

12. Vorrichtung mit mehreren Kompartimenten, in der ein erstes Kompartiment eine Färbezusammensetzung gemäß einem der Ansprüche 1 bis 9 enthält und ein zweites Kompartiment ein oder mehrere Oxidationsmittel enthält.

13. Verwerdung einer Zusammensetzung gemäß einem der Ansprüche 1 bis 10 zum Oxidationsfärben von Keratinfasern.

## Claims

1. Composition for dyeing keratin fibers, comprising, in a suitable medium:
• at least a first oxidation base chosen from 2,3-diamino-6,7-dihydro-1H,5H-pyrazolo[1,2-a]pyrazol-1-one and addition salts thereof;
• at least a second oxidation base chosen from 4,5-diamino-1-(β-hydroxyethyl)pyrazole and addition salts thereof; and
• at least one coupler chosen from 2-chloro-6-methyl-3-aminophenol and addition salts thereof.

2. Composition according to Claim 1, in which the molar ratio of the concentration of 2-chloro-6-methyl-3-aminophenol, or an addition salt thereof, to the total of the concentrations of the first and second oxidation bases is less than or equal to 1.

3. Composition according to either of Claims 1 and 2, comprising one or more additional oxidation base(s) chosen from para-aminophenols and para-phenylenediamines.

4. Composition according to Claim 3, in which the para-aminophenol(s) is (are) chosen from para-aminophenol and 3-methyl-4-aminophenol, and addition salts thereof.

5. Composition according to Claim 3, in which the para-phenylenediamine(s) is (are) chosen from para-phenylenediamine, para-toluenediamine and N,N-bis(β-hydroxyethyl)-para-phenylenediamine, and addition salts thereof.

6. Composition according to any one of the preceding claims, comprising one or more additional coupler(s).

7. Composition according to Claim 6, in which the additional coupler(s) is (are) chosen from 2-methyl-5-aminophenol and 2-methyl-5-(β-hydroxyethylamino)phenol, and addition salts thereof.

8. Composition according to any one of the preceding claims, in which the oxidation base(s) is (are each) present in an amount of between 0.001% and 10% by weight approximately, relative to the total weight of the composition.

9. Composition according to any one of the preceding claims, in which the coupler(s) is (are each) present in an amount of between 0.001% and 10% by weight approximately, relative to the total weight of the composition.

10. Composition according to any one of the preceding claims, also comprising one or more oxidizing agent(s).

11. Process for dyeing keratin fibers, in which a composition as defined in any one of Claims 1 to 9 is applied to the keratin fibers in the presence of one or more oxidizing agent(s) for a period of time that is sufficient to develop the desired colouration.

12. Multi-compartment device in which a first compartment contains a dye composition as defined in any one of Claims 1 to 9, and a second compartment contains one or more oxidizing agent(s).

13. Use of a composition as defined in any one of Claims 1 to 10, for the oxidation dyeing of keratin fibers.
